# FASCICULE DE BREVET EUROPEEN

(11) **EP 1 263 352 B1**
(45) Date de publication et mention de la délivrance du brevet: **15.12.2004**
(21) Numéro de dépôt: 01913926.0
(22) Date de dépôt: 02.03.2001
(51) Int. Cl.: A61F 2/44

(54) **PROTHESE DISCALE POUR VERTEBRES CERVICALES**
BANDSCHEIBENPROTHESE FÜR HALSWIRBELSÄULE
DISC PROSTHESIS FOR CERVICAL VERTEBRA

(30) Priorité: 03.03.2000 FR 0002791
(43) Date de publication de la demande: 11.12.2002
(73) Titulaire: SCIENT'X, 78284 Guyancourt (FR)
(72) Inventeur: RAMADAN, Aymen, CH-1227 Carouge (CH); BUHLER, Markus, CH-8610 Uster (CH)
(74) Mandataire: Thibault, Jean-Marc
(86) Numéro de dépôt international: PCT/FR2001/000622
(87) Numéro de publication internationale: WO 2001/064140

(56) Documents cités:
- EP-A- 0 699 426
- FR-A- 2 694 882
- FR-A- 2 718 635
- US-A- 6 019 792

## Description

L'objet de l'invention concerne une prothèse discale pour vertèbres cervicales, destinée à être substituée au disque fibre-cartilagineux assurant la liaison entre les vertèbres cervicales de la colonne vertébrale.

Il est connu qu'un disque intervertébral peut subir des altérations, telles que tassement, déformation, déplacement ou usure et plus, généralement, une dégénérescence associée à des contraintes mécaniques qui lui sont appliquées et conduisent à une destruction anatomique et fonctionnelle du disque et du segment vertébral. Cette altération discale modifie le comportement mécanique du disque et aboutit à une diminution de la hauteur de l'espace intersomatique, laquelle entraîne une perturbation de l'ensemble fonctionnel articulaire. Il en résulte une instabilité induisant, en particulier, une réaction arthrosique, source de douleurs et de processus ostéophytiques.

Il a donc été proposé de remplacer le disque déficient par un disque artificiel dont différents types de réalisation ont été envisagés. Ainsi, il est connu, par exemple par le brevet **FR 2 718 635**, une prothèse discale pour vertèbres cervicales comprenant une première et une deuxième plaques destinées à être fixées à des vertèbres cervicales voisines. Cette prothèse comporte également une rotule d'articulation interposée entre les deux plaques montées en position superposée. La rotule est composée d'une calotte sphérique réalisée en un matériau synthétique, tel que polyéthylène, montée sur l'une des plaques et destinée à coopérer avec une cupule sphérique aménagée sur l'autre plaque réalisée en un matériau métallique, tel qu'en titane par exemple.

Si une telle prothèse cervicale permet de restaurer une hauteur convenable à l'espace intersomatique, la rotule d'articulation de cette prothèse présente des frottements importants la rendant sensible à l'usure, la conséquence de cette usure étant que cette prothèse ne donne pas entière satisfaction en raison de sa relative instabilité, notamment lors des mouvements de flexion.

Il est connu, par ailleurs, notamment par le brevet **US 5 562 738**, une prothèse discale pour vertèbres lombaires comportant une première et une deuxième plaques de fixation aux vertèbres voisines, réalisées en un matériau métallique, tel qu'en titane. Entre les plaques, est interposée une rotule d'articulation comportant un premier insert monté sur l'une des plaques et composé d'une calotte sphérique coopérant avec une cupule sphérique d'un deuxième insert monté sur l'autre plaque. Les inserts sont réalisés en un matériau céramique biocompatible présentant des caractéristiques tribologiques améliorées, notamment en ce qui concerne sa résistance à l'usure.

Toutefois, une telle prothèse discale pour vertèbres lombaires, ne se trouve pas adaptée pour remplacer le disque des vertèbres cervicales, dans la mesure où une telle prothèse ne permet pas aux vertèbres cervicales de retrouver leur mobilité naturelle. Il s'avère également que la rotule d'articulation présente une forme de réalisation relativement difficile à mener à bien et sensible aux phénomènes de brisure ou de fêlure, réduisant ainsi la durée de vie de la prothèse.

L'objet de la présente invention vise donc à remédier aux inconvénients de l'état de la technique en proposant une prothèse discale pour vertèbres cervicales conçue pour présenter une durée de vie relativement longue, en étant pratiquement insensible aux phénomènes d'usure et de brisure, tout en étant adaptée pour autoriser une mobilité physiologique entre les deux vertèbres cervicales instrumentées.

Pour atteindre un tel objectif, la prothèse selon l'invention est du type comportant :
- une première et une deuxième plaques destinées à être fixées à des vertèbres cervicales voisines,
- et une rotule d'articulation interposée entre les deux plaques montées en position superposée, la rotule étant composée d'une calotte sphérique coopérant avec une cupule sphérique.
   Selon l'invention :
   - la calotte sphérique est aménagée sur un premier insert, tandis que la cupule sphérique est aménagée sur un deuxième insert,
   - chaque insert est réalisé en un matériau céramique et possède une base de section droite transversale circulaire,
   - l'un des inserts est monté sur la première plaque, tandis que l'autre des inserts est monté sur la deuxième plaque, de manière que le centre d'articulation de la rotule se trouve sensiblement centré par rapport aux bords des plaques, en vue d'être centré dans le plan sagittal et dans le plan frontal des vertèbres,
   - la cupule sphérique possède une surface de contact au moins égale à celle de la calotte sphérique et se trouve raccordée, par une moulure annulaire, à la base de l'insert,
   - et la plaque munie de l'insert présentant la calotte sphérique comporte un dégagement annulaire pour autoriser le débattement de la moulure annulaire de la cupule sphérique lors du mouvement des plaques.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.
La **fig. 1** est une coupe en élévation d'un premier exemple de réalisation d'une prothèse conforme à l'invention.
La **fig. 2** est une vue intérieure prise sensiblement selon la flèche **F**_{**1**}**,** d'une première plaque faisant partie de la prothèse selon l'invention.
La **fig. 3** est une vue intérieure prise sensiblement selon la flèche **F**_{**2**}**,** d'une deuxième plaque faisant partie de la prothèse selon l'invention.
La **fig. 4** est une vue de dessus avec une coupe partielle de la prothèse illustrée à la **fig. 1.**
La **fig. 5** est une vue en élévation d'un deuxième exemple de réalisation d'une prothèse selon l'invention.
La **fig. 6** est une vue de dessus de la prothèse illustrée à la **fig. 5.**

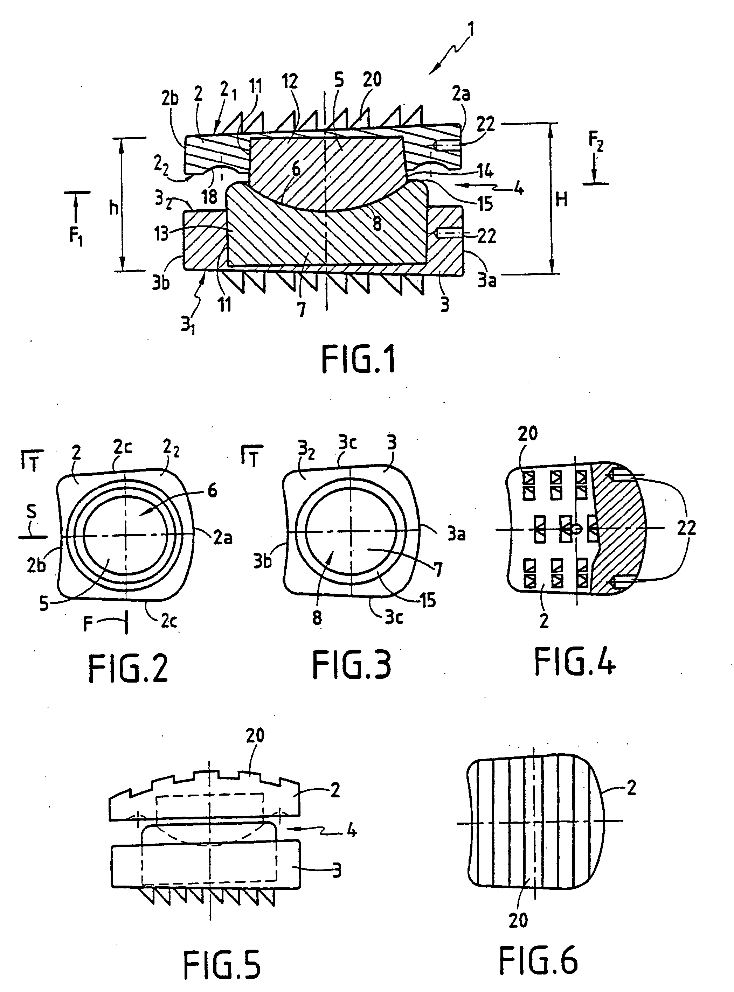

Tel que cela ressort plus précisément des **fig. 1** à **4,** l'objet de l'invention concerne une prothèse discale **1** destinée à être implantée à la place d'un disque, entre deux vertèbres cervicales adjacentes. La prothèse cervicale **1** selon l'invention comporte une première plaque **2**, dite supérieure dans l'exemple illustré, et une deuxième plaque **3**, dite inférieure. Les plaques **2** et **3** sont destinées à être fixées à des vertèbres cervicales voisines et présentent chacune une face externe respectivement **2**_{**1**}**, 3**_{**1**} de dimensions sensiblement similaires et adaptée pour épouser approximativement le contour des surfaces articulaires associées. Chaque plaque **2, 3** comporte également une face interne, respectivement **2**_{**2**}**, 3**_{**2**} s'étendant en vis-à-vis l'une de l'autre. Chaque plaque **2, 3** présente une forme générale parallélèpipédique en possédant un bord postérieur respectivement **2a, 3a** s'étendant à l'opposé d'un bord antérieur **2b, 3b,** respectivement. Le bord antérieur **2b, 3b** de chaque plaque **2, 3** est relié au bord postérieur **2a, 3a** respectif, à l'aide de deux bords latéraux **2c, 3c** opposés l'un de l'autre. De préférence, les bords **2a, 2b, 2c** et **3a, 3b, 3c** présentent un profil droit et sont reliés entre eux par des congés de raccordement. De préférence, le bord postérieur **2a**, **3a** de chaque plaque présente, dans un plan transversal **T**, un profil convexe, tandis que le bord antérieur **2b**, **3b** présente un profil concave.

La prothèse cervicale **1** selon l'invention comporte également une rotule d'articulation **4** interposée entre les deux plaques **2** et **3** qui se trouvent montées en position superposée. La rotule d'articulation **4** est constituée par un premier insert **5** présentant une calotte sphérique **6** et par un deuxième insert **7** présentant une cupule sphérique **8** coopérant avec la calotte sphérique **6.** Chaque insert **5, 7** est destiné à être monté dans un logement **11,** de préférence borgne, réalisé à partir de la face interne **2**_{**2**}**, 3**_{**2**} de chaque plaque **2** et **3.** Chaque insert **5, 7** présente une forme générale de révolution et possède respectivement une base **12, 13** de section droite transversale circulaire dont l'une des extrémités est aménagée pour présenter la calotte sphérique **6** ou la cupule sphérique **8**. La section droite transversale de la base **12, 13** de chaque insert **5, 7** est constante ou, de préférence, décroissante à partir de la calotte sphérique **6** ou de la cupule sphérique **8.** Selon cette dernière forme préférée de réalisation, chaque logement de réception **11** présente une forme conjuguée à celle de l'insert **5, 7** pour permettre un assemblage conique des inserts **5**, **7** sur les plaques **2**, **3**. Bien entendu, il peut être envisagé un type d'assemblage différent pour les inserts, par exemple par collage ou par sertissage. Selon une forme de réalisation, il pourrait être prévu de monter au fond du logement borgne **11**, un élément d'amortissement destiné à être interposé entre un insert et la plaque, de manière à amortir les sollicitations axiales exercées sur la prothèse.

Selon une forme préférée de réalisation, l'insert **5** pourvu de la calotte sphérique **6** est monté sur la plaque dite supérieure **2**, tandis que l'insert **7** pourvu de la cupule sphérique **8** est monté sur la plaque inférieure **3**. Une telle disposition permet à la rotule d'articulation **4** de mieux encaisser les efforts qu'elle subit.

La calotte sphérique **6** est délimitée par une surface de contact présentant un rayon de courbure qui est égal au rayon de courbure de la surface de contact délimitant la cupule sphérique **8**, de manière à former une articulation à rotule. La calotte sphérique **6** est reliée à la base **12** du premier insert **5** par l'intermédiaire d'un congé de raccordement **14**, tandis que la cupule sphérique **8** est raccordée à la base **13** du deuxième insert **7** par l'intermédiaire d'un congé de raccordement formant une moulure annulaire **15**. Selon une caractéristique avantageuse de l'invention, la cupule sphérique **8** possède une surface de contact au moins égale à celle de la calotte sphérique **6**, afin d'obtenir une bonne tenue mécanique entre la calotte sphérique **6** et la cupule sphérique **8**. En d'autres termes, la base **13** du deuxième insert **7** pourvu de la cupule sphérique **8**, présente donc une section droite transversale circulaire supérieure à la section droite transversale de la base **12** du premier insert **5** pourvu de la calotte sphérique **6**, en raison de la présence de la moulure annulaire **15**.

Selon une autre caractéristique avantageuse de l'invention, la plaque **2** munie de premier insert **5** présentant la calotte sphérique **6**, comporte un dégagement annulaire **18** bordant le logement **11** pour autoriser le débattement de la moulure annulaire **15** de la cupule sphérique **5,** lors des mouvements des plaques **2, 3.** Bien entendu, le premier insert **5** est monté sur la plaque **2,** de manière à s'étendre en saillie par rapport à la face interne **2**_{**2**} pour permettre qu'il coopère avec la cupule sphérique **8.** De même, le deuxième insert **7** est monté pour s'étendre en saillie par rapport à la face interne **3**_{**2**} de la plaque pour obtenir un débattement angulaire suffisant entre les plaques sans que celles-ci viennent en contact l'une contre l'autre.

Selon une caractéristique avantageuse de l'invention, les inserts **5**, **7** sont réalisés en un matériau céramique. De préférence, chaque insert **5**, **7** est réalisé en un matériau céramique de dureté différente. Par exemple, le deuxième insert **7** muni de la cupule sphérique **8** est réalisé en oxyde de zirconium (ZrO₂), tandis que le premier insert **5** muni de la calotte sphérique **6** est réalisé en oxyde d'aluminium (Al₂O₃).

Selon une autre caractéristique avantageuse de l'invention, les inserts **5**, **7** sont montés sur les plaques **2** et **3**, de manière que le centre d'articulation de la rotule **4** se trouve sensiblement centré par rapport, d'une part, aux bords latéraux **2c, 3c** des plaques pour être centré dans le plan sagittal ou antéro-postérieur **S** et, d'autre part, aux bords antérieur **2a**, **3a** et postérieur **2b**, **3b** des plaques, afin d'être centré dans le plan frontal **F** des vertèbres. Une telle disposition centrée du centre de rotation de la rotule **4** permet à la prothèse **1** de retrouver les mouvements naturels du disque intervertébral des vertèbres cervicales.

Grâce au dimensionnement approprié de la rotule **4** et de la position de son centre d'articulation, tel que défini ci-dessus, les efforts appliqués sur les surfaces en contact sont limités. Il est à noter que les surfaces en contact correspondent à la calotte sphérique **6** et à la cupule sphérique **8**, pour un débattement angulaire égal ou inférieur à 10 degrés, de sorte que les frottements apparaissent toujours entre les inserts **5**, **7**, c'est-à-dire entre des surfaces réalisées en matière céramique. Il en résulte une usure réduite des inserts. La limitation de l'amplitude des mouvements est obtenue par la mise en contact des plaques **2**, **3** entre elles. Par ailleurs, l'emboîtement de la calotte sphérique **6** à l'intérieur de la cupule sphérique 8 permet d'obtenir une stabilité pour l'articulation **4** tout en lui assurant une mobilité tridimensionnelle convenable pratiquement identique à celle d'un disque naturel.

Les plaques **2**, **3** peuvent être avantageusement exécutées en titane et les surfaces de contact avec les plateaux vertébraux des vertèbres, à savoir les faces externes **2**_{**1**}**, 3**_{**1**}**,** sont de préférence recouvertes d'hydroxy apatite ou de titane à effet de surface par exemple, afin d'améliorer l'ancrage entre la prothèse et l'os adjacent.

Il est à noter qu'il peut être prévu des plaques **2**, **3** de formes différentes adaptées à différents morphotypes possibles des corps vertébraux. Ainsi, il peut être prévu que la ou les plaques **2**, **3** peuvent présenter des hauteurs différentes pour permettre de s'adapter à la hauteur de l'espace intersomatique à rétablir. Par ailleurs, comme dans l'exemple illustré à la **fig. 1,** il peut être prévu que les faces externes **2**_{**1**}**, 3**_{**1**} des plaques **2** et **3** présentent un profil plan équipé ou non d'éléments d'ancrage **20** dans les vertèbres. Dans l'exemple illustré aux **fig. 1** et **4,** chaque face externe **2**_{**1**}**, 3**_{**1**} possède des crans d'ancrage **20.** Dans l'exemple illustré aux **fig. 5, 6,** la face externe **2**_{**1**} de la première plaque **2** comporte, en tant qu'élément d'ancrage **20,** des crans parallèles entre eux et au plan frontal **F**. Il peut être envisagé que la plaque supérieure **2** présente un profil convexe selon le plan sagittal, comme illustré à la **fig. 5.** Selon une autre caractéristique de l'invention, l'une et/ou l'autre des faces externes **2**_{**1**}**, 3**_{**1**} des plaques **2, 3** est pourvue de deux picots d'ancrage non représentés et suffisamment longs pour traverser chaque plateau des vertèbres pour éviter un glissement de la cage.

Tel que cela apparaît plus précisément aux **fig. 2** et **4**, chaque plaque **2, 3** est pourvue, sur ses bords postérieurs **2a**, **3a**, de deux trous de positionnement **22** pour les embouts d'un outil assurant la préhension simultanée des deux plaques. Il est à noter que dans cette position, telle qu'illustrée à la **fig. 1,** les plaques **2, 3** forment un angle dans le plan sagittal **S**, pour faciliter l'introduction de la cage dans l'espace intersomatique. En effet, il apparaît que la hauteur h de la prothèse **1** au niveau de sa face avant définie par les bords antérieurs **2b**, **3b**, est inférieure à sa hauteur **H** de sa partie arrière définie par les bords postérieurs **2a**, **3a**. Selon une caractéristique préférée de réalisation, les trous de positionnement **22** d'une même plaque convergent entre eux pour faciliter le retrait de l'outil de positionnement.

L'invention n'est pas limitée aux exemples décrits et représentés, car diverses modifications peuvent y être apportées sans sortir de son cadre.

## Revendications

1. Prothèse discale pour vertèbres cervicales, du type comportant :
- une première **(2)** et une deuxième **(3)** plaques destinées à être fixées à des vertèbres cervicales voisines,
- et une rotule d'articulation **(4)** interposée entre les deux plaques **(2, 3)** montées en position superposée, la rotule étant composée d'une calotte sphérique **(6)** coopérant avec une cupule sphérique **(8)**, **caractérisée en ce que** :
• la calotte sphérique **(6)** est aménagée sur un premier insert **(5),** tandis que la cupule sphérique **(8)** est aménagée sur un deuxième insert **(7),**
• chaque insert **(5, 7)** est réalisé en un matériau céramique et possède une base **(12, 13) de** section droite transversale circulaire,
• l'un des inserts **(5, 7)** est monté sur la première plaque **(2),** tandis que l'autre des inserts **(5, 7)** est monté sur la deuxième plaque **(3),** de manière que le centre d'articulation de la rotule **(4)** se trouve sensiblement centré par rapport aux bords des plaques, en vue d'être centré dans le plan sagittal **(S)** et dans le plan frontal **(F)** des vertèbres,
• la cupule sphérique **(8)** possède une surface de contact au moins égale à celle de la calotte sphérique **(6)** et se trouve raccordée, par une moulure annulaire **(15)**, à la base de l'insert,
• et la plaque munie de l'insert présentant la calotte sphérique **(8)** comporte un dégagement annulaire **(18)** pour autoriser le débattement de la moulure annulaire **(15)** de la cupule sphérique **(8)** lors des mouvements des plaques.

2. Prothèse discale selon la revendication 1, **caractérisée en ce que** chaque plaque **(2, 3)** est aménagée pour présenter un logement borgne **(11)** de réception pour un insert.

3. Prothèse discale selon les revendications 1 et 2, **caractérisée en ce que** chaque insert **(5, 7)** possède une base **(12, 13)** dont la section droite transversale circulaire décroît à partir de la calotte sphérique **(6)** ou de la cupule sphérique **(8)**, et se trouve adaptée au logement borgne (**11**) de profil conjugué.

4. Prothèse discale selon la revendication 1, **caractérisée en ce que** chaque insert **(5, 7)** est réalisé en un matériau céramique de dureté différente.

5. Prothèse discale selon la revendication 4, **caractérisée en ce que** l'insert muni de la cupule sphérique **(8)** est réalisé en oxyde de zirconium, tandis que l'insert muni de la calotte sphérique **(6)** est réalisé en oxyde d'aluminium.

6. Prothèse discale selon la revendication 2, **caractérisée en ce qu'**elle comporte un élément d'amortissement monté dans le fond du logement borgne **(11)** destiné à être interposé entre l'insert et la plaque.

7. Prothèse discale selon la revendication 1, **caractérisée en ce que** la plaque **(2)** équipée de la calotte sphérique **(6)** s'étend au-dessus de la plaque **(3)** équipée de la cupule sphérique **(8).**

8. Prothèse discale selon la revendication 1 ou 7, **caractérisée en ce que** la plaque **(2)** s'étendant au-dessus de l'autre plaque **(3)** présente une face externe supérieure présentant un profil convexe selon le plan sagittal **(S).**

9. Prothèse discale selon la revendication 1, **caractérisée en ce que** chaque plaque **(2, 3)** possède une face externe de profil plan.

10. Prothèse discale selon la revendication 8 ou 9, **caractérisée en ce que** la face externe de l'une et/ou de l'autre des plaques est pourvue de crans d'ancrage **(20)** dans les vertèbres.

11. Prothèse discale selon la revendication 10, **caractérisée en ce que** les crans d'ancrage **(20)** sont constitués par des nervures parallèles entre elles et par rapport aux bords postérieurs des plaques.

12. Prothèse discale selon la revendication 8 ou 9, **caractérisée en ce que** la face externe (**2**_{**1**}**, 3**_{**1**}) de l'une et/ou l'autre des plaques est pourvue deux picots permettant d'éviter le glissement de la cage.

13. Prothèse discale selon la revendication 1, **caractérisée en ce que** chaque plaque **(2, 3)** est pourvue sur ses bords postérieurs, de deux trous de positionnement **(22)** pour les embouts d'un outil assurant la préhension simultanée des deux plaques qui dans cette position, forment dans le plan sagittal un cône d'introduction.

14. Prothèse discale selon la revendication 1, **caractérisée en ce que** chaque plaque **(2, 3)** présente un bord antérieur **(2b, 3b)** de profil concave dans le plan transversal.

## Patentansprüche

1. Bandscheibenprothese für Halswirbelsäulen, die folgendes umfaßt:
- eine erste (2) und eine zweite (3) Platte, die dazu bestimmt sind, an benachbarten Halswirbelsäulen befestigt zu werden,
- und ein Kugelgelenk (4), das zwischen den beiden Platten (2, 3) angeordnet ist, welche übereinander liegend befestigt sind, wobei das Kugelgelenk aus einer kugelförmigen Kappe (6) besteht, die mit einer kugelförmigen Haube (8) zusammenarbeitet, **dadurch gekennzeichnet, daß**
- die kugelförmige Kappe (6) auf einem ersten Einschub (5) angeordnet ist, während die kugelförmige Haube (8) auf einem zweiten Einschub (7) angeordnet ist,
- jeder Einschub (5, 7) aus einem keramischen Material hergestellt ist und eine Basis (12, 13) mit einem kreisförmigen Querschnitt aufweist,
- einer der Einschübe (5, 7) auf der ersten Platte (2) befestigt ist, während der andere der Einschübe (5, 7) auf der zweiten Platte (3) befestigt ist, so daß das Gelenkzentrum des Kugelgelenks (4) im wesentlichen in Bezug auf die Ränder der Platten zentriert ist, um auf der Sagittalebene (S) und der Frontalebene (F) der Wirbel zentriert zu sein,
- die kugelförmige Haube (8) eine Kontaktoberfläche aufweist, die jener der kugelförmigen Kappe (6) mindestens gleich ist und durch eine ringförmige Leiste (15) mit der Basis des Einschubes verbunden ist,
- die Platte, die mit dem Einschub versehen ist, welcher die kugelförmige Haube (8) aufweist, eine ringförmige Ausnehmung (18) umfaßt, um während den Bewegungen der Platten die Durchfederung der ringförmigen Leiste (15) von der kugelförmigen Haube (8) zu ermöglichen.

2. Bandscheibenprothese nach Anspruch 1, **dadurch gekennzeichnet, daß** jede Platte (2, 3) so angeordnet ist, daß sie für einen Einschub eine einseitig offene Aufnahmeaussparung (11) aufweist.

3. Bandscheibenprothese nach Anspruch 1 und 2, **dadurch gekennzeichnet, daß** jeder Einschub (5, 7) eine Basis (12, 13) aufweist, deren kreisförmiger Querschnitt ausgehend von der kugelförmigen Kappe (6) oder der kugelförmigen Haube (8) abnimmt und an die einseitig offene Aufnahmeaussparung (11) mit konjugiertem Profil angepaßt ist.

4. Bandscheibenprothese nach Anspruch 1, **dadurch gekennzeichnet, daß** jeder Einschub (5, 7) aus einem keramischen Material mit unterschiedlicher Härte hergestellt ist.

5. Bandscheibenprothese nach Anspruch 4, **dadurch gekennzeichnet, daß** der Einschub, der mit der kugelförmigen Haube (8) versehen ist, aus Zirkoniumoxid besteht, während der Einschub, der mit der kugelförmigen Kappe (6) versehen ist, aus Aluminiumoxid besteht.

6. Bandscheibenprothese nach Anspruch 2, **dadurch gekennzeichnet, daß** die Prothese ein Dämpfungselement aufweist, welches am Boden der einseitig offenen Aufnahmeaussparung (11) angeordnet ist, um zwischen dem Einschub und der Platte eingefügt zu werden.

7. Bandscheibenprothese nach Anspruch 1, **dadurch gekennzeichnet, daß** sich die Platte (2), die mit der kugelförmigen Kappe (6) versehen ist, oberhalb der Platte (3) erstreckt, die mit der kugelförmigen Haube (8) versehen ist.

8. Bandscheibenprothese nach Anspruch 1 oder 7, **dadurch gekennzeichnet, daß** die Platte (2), die sich oberhalb der anderen Platte (3) erstreckt, eine obere Außenfläche aufweist, die ein konvexes Profil entlang der Sagittalebene (S) aufweist.

9. Bandscheibenprothese nach Anspruch 1, **dadurch gekennzeichnet, daß** jede Platte (2, 3) eine Außenfläche mit ebenem Profil aufweist.

10. Bandscheibenprothese nach Anspruch 8 oder 9, **dadurch gekennzeichnet, daß** die Außenfläche der einen und/oder der anderen Platte in den Wirbeln mit Verankerungskerben (20) versehen ist.

11. Bandscheibenprothese nach Anspruch 10, **dadurch gekennzeichnet, daß** die Verankerungskerben (20) aus Rillen bestehen, die zueinander und in Bezug auf die hinteren Ränder der Platten parallel sind.

12. Bandscheibenprothese nach Anspruch 8 oder 9, **dadurch gekennzeichnet, daß** die Außenfläche (2₁, 3₁) der einen und/oder der anderen Platte mit zwei Spitzen versehen ist, welche es ermöglichen, das Verschieben des Gehäuses zu vermeiden.

13. Bandscheibenprothese nach Anspruch 1, **dadurch gekennzeichnet, daß** jede Platte (2, 3) auf ihren hinteren Rändern mit zwei Positionierungsöffnungen (22) für den Ansatz eines Werkzeuges versehen ist, welche das gleichzeitige Erfassen der zwei Platten gewährleisten, die in dieser Position auf der Sagittalebene einen Einführungskegel bilden.

14. Bandscheibenprothese nach Anspruch 1, **dadurch gekennzeichnet, daß** jede Platte (2, 3) einen vorderen Rand (2b, 3b) mit einem konkaven Profil auf der Querebene aufweist.

## Claims

1. A disk prosthesis for cervical vertebrae, the prosthesis being of the type comprising:
- first and second plates (2, 3) designed to be fixed to adjacent cervical vertebrae; and
- a ball joint (4) interposed between the two plates (2, 3) mounted in a superposed position, the joint being constituted by a spherical cap (6) co-operating with a spherical cup (8), **characterised in that**:
• the spherical cap (6) is provided on a first insert (5), while the spherical cup (8) is provided on a second insert (7);
• each insert (5, 7) is made of a ceramic material and possesses a base (12, 13) of circular right cross-section;
• one of the inserts (5, 7) is mounted on the first plate (2) while the other insert (5, 7) is mounted on the second plate (3) in such a manner that the centre of rotation of the joint (4) lies substantially centred relative to the edges of the plates so as to be centred in the sagittal plane (S) and in the frontal plane (F) of the vertebrae;
• the spherical cup (8) possesses a contact area that is not less that the contact area of the spherical cap (6) and is connected via an annular moulding (15) to the base of the insert; and
• the plate provided with the insert having the spherical cap (6) has an annular setback (18) to leave clearance for the annular moulding (15) of the spherical cup (8) during movements of the plates.

2. A disk prosthesis according to claim 1, **characterised in that** each plate (2, 3) is organised to present a blind housing (11) for receiving an insert.

3. A disk prosthesis according to claim 1 or claim 2, **characterised in that** each insert (5, 7) possesses a base (12, 13) whose circular right cross-section tapers from the spherical cap (6) or the spherical cup (8), and matches the blind housing (11) of complementary profile.

4. A disk prosthesis according to claim 1, **characterised in that** each insert (5, 7) is made of ceramic materials of different hardnesses.

5. A disk prosthesis according to claim 4, **characterised in that** the insert fitted with the spherical cup (8) is made of zirconium oxide, while the insert fitted with the spherical cap (6) is made of aluminium oxide.

6. A disk prosthesis according to claim 2, **characterised in that** it includes a damping element mounted in the end of the blind housing (11) so as to be interposed between the insert and the plate.

7. A disk prosthesis according to claim 1, **characterised in that** the plate (2) fitted with the spherical cap (6) extends over the plate (3) fitted with the spherical cup (8).

8. A disk prosthesis according to claim 1 or claim 7, **characterised in that** the plate (2) extending over the other plate (3) has a top outer face that presents a profile that is convex in the sagittal plane (S).

9. A disk prosthesis according to claim 1, **characterised in that** each plate (2, 3) possesses an outer face of profile that is plane.

10. A disk prosthesis according to claim 8 or claim 9, **characterised in that** the outer face of at least one of the plates is provided with anchoring notches (20) for anchoring in the vertebrae.

11. A disk prosthesis according to claim 10, **characterised in that** the anchoring notches (20) are constituted by ribs that are parallel to one another and parallel to the posterior edges of the plates.

12. A disk prosthesis according to claim 8 or claim 9, **characterised in that** the outer face (2₁, 3₁) of at least one of the plates is provided with two studs for preventing the cage from sliding.

13. A disk prosthesis according to claim 1, **characterised in that** each plate (2, 3) is provided on its posterior edges with two positioning holes (22) for the studs of a tool for holding both plates simultaneously, which plates, when in this position, form an insertion cone in the sagittal plane.

14. A disk prosthesis according to claim 1, **characterised in that** each plate (2, 3) has an anterior edge (2b, 3b) of profile that is concave in the transverse plane.
